(19) Europäisches Patentamt
European Patent Office
Office européen des brevets

(11) EP 1 411 069 B1

(12) FASCICULE DE BREVET EUROPEEN

(45) Date de publication et mention de la délivrance du brevet:
**03.11.2010 Bulletin 2010/44**

(21) Numéro de dépôt: **03292382.3**

(22) Date de dépôt: **26.09.2003**

(51) Int Cl.:
***A61K 8/90*** *(2006.01)* ***A61K 8/81*** *(2006.01)*
***A61Q 1/04*** *(2006.01)* ***A61Q 1/10*** *(2006.01)*
***A61Q 3/02*** *(2006.01)* ***A61K 8/26*** *(2006.01)*
***A61K 8/39*** *(2006.01)* ***A61K 8/88*** *(2006.01)*
***A61K 8/89*** *(2006.01)* ***A61K 8/92*** *(2006.01)*
***A61Q 1/02*** *(2006.01)* ***A61Q 1/06*** *(2006.01)*

(54) **Polymères séquencés et compositions cosmétiques contenant de tels polymères**

Blockcopolymere und kosmetische Zusammensetzungen mit solchen Polymeren

Block copolymers and cosmetic compositions containing such polymers

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211949**
**21.05.2003 FR 0306121**

(43) Date de publication de la demande:
**21.04.2004 Bulletin 2004/17**

(73) Titulaire: **L'Oréal**
**75008 Paris (FR)**

(72) Inventeurs:
- **Lion, Bertrand**
**95270 Luzarches (FR)**
- **Martin, Nathalie**
**95270 Viarnes (FR)**
- **Toumi, Béatrice**
**91370 Verrieres le Buisson (FR)**

(74) Mandataire: **Boulard, Denis**
**L'Oréal**
**D.I.P.I.**
**25-29 Quai Aulagnier**
**92600 Asnières (FR)**

(56) Documents cités:
**EP-A- 1 024 184** **EP-A- 1 043 344**
**DE-A- 10 029 697** **FR-A- 2 809 306**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a trait à de nouveaux polymères de structure spécifique.

La présente invention se rapporte également à des compositions cosmétiques comprenant de tels polymères.

Divers types de polymères sont conventionnellement utilisés dans les compositions cosmétiques en raison des diverses propriétés qu'ils peuvent apporter à ces compositions.

Ils sont par exemple utilisés dans des compositions de maquillage ou de soin de la peau, des lèvres ou des phanères telles que des vernis à ongles ou des compositions pour les cheveux, tels ceux décrits dans FR-A-2 809 306.

Cependant, en utilisant au sein d'une même composition deux polymères incompatibles, c'est à dire non miscibles dans un même solvant, le formulateur est confronté, du fait de l'incompatibilité des polymères, à des problèmes de séparation de phases, voire de décantation, et de manière générale à l'obtention d'une composition non homogène. Ces problèmes ne pouvaient être jusqu'ici résolus que par la présence dans la composition d'un composé permettant de compatibiliser les polymères entre eux.

Le but de la présente invention est de proposer un polymère qui, lorsqu'il est inclus dans une composition, en particulier une composition cosmétique, permette que cette composition ne présente pas les inconvénients, limitations, défauts et désavantages des compositions de l'art antérieur.

Ce but est atteint, conformément à la présente invention, grâce à un polymère, dit polymère séquencé, comprenant au moins une première séquence et au moins une deuxième séquence incompatibles l'une avec l'autre et ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence, ledit polymère séquencé ayant un indice de polydispersité I supérieur à 2,5; ladite première séquence étant choisie parmi :

a) une séquence ayant une Tg supérieure ou égale à 40°C,
b) une séquence ayant une Tg inférieure ou égale à 20°C,
c) une séquence ayant une Tg comprise entre 20 et 40°C, et

ladite deuxième séquence étant choisie dans une catégorie a), b) ou c) différente de la première séquence; ledit segment intermédiaire étant un polymère statistique.

Par "au moins" une séquence, on entend une ou plusieurs séquences. Par "séquences incompatibles l'une avec l'autre", on entend que le mélange formé du polymère correspondant à la première séquence et du polymère correspondant à la deuxième séquence, n'est pas miscible dans le solvant de polymérisation majoritaire en poids du polymère séquencé, à température ambiante (25°C) et pression atmosphérique ($10^5$ Pa), pour une teneur du mélange de polymères supérieure ou égale à 5% en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé ± 15%.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.

Bien entendu, dans le cas d'une polymérisation réalisée dans un solvant unique, ce dernier est le solvant majoritaire.

Le segment intermédiaire est une séquence comprenant au moins un monomère constitutif de la première séquence et au moins un monomère constitutif de la deuxième séquence du polymère permet de "compatibiliser" ces séquences.

En incorporant ces nouveaux polymères dans des compositions cosmétiques, la demanderesse a découvert que certains de ces polymères décrits plus en détail ci-dessous, avaient des propriétés cosmétiques très intéressantes. De manière générale, ces polymères peuvent être incorporés dans des compositions à une teneur élevée en matières sèche, typiquement supérieure à 10% en poids par rapport au poids total de la composition et présentent une facilité de formulation. Utilisés dans des produits pour cheveux, ils en améliorent à la fois le pouvoir coiffant et la souplesse. Ils augmentent la résistance aux chocs des vernis à ongles et améliorent la tenue d'une grande variété de compositions de maquillage sans provoquer chez l'utilisateur un sentiment d'inconfort.

L'invention a également pour objet une composition cosmétique comprenant un tel polymère.

Un autre objet de l'invention est aussi un procédé cosmétique de maquillage ou de soin des matières kératiniques comprenant l'application sur les matières kératiniques, d'une composition cosmétique selon l'invention.

L'invention se rapporte e ncore à l'utilisation du polymère selon l'invention dans une composition cosmétique, comme agent pour améliorer la tenue de ladite composition.

L'invention se rapporte enfin à l'utilisation du polymère selon l'invention dans une composition présentant des propriétés de tenue améliorées.

Le polymère séquence de la composition selon l'invention est avantageusement un polymère éthylénique séquence linéaire filmogène

Par polymère "éthylénique" , on entend un polymère obtenu par polymérisation de monomères comprenant une insaturation éthylénique.

Par polymère "séquencé", on entend un polymère comprenant au moins 2 séquences distinctes, de préférence au moins 3 séquences distinctes.

Le polymère est un polymère à structure linéaire. Par opposition, un polymère a structure non linéaire est, par exemple, un polymère à structure ramifiée, en étoile, greffée, ou autre.

Par polymère "filmogène", on entend un polymère apte à former à lui seul ou en présence d'un agent auxiliaire de filmification, un film continu et adhérent sur un support, notamment sur les matières kératiniques.

De façon préférentielle, le polymère selon l'invention ne comprend p as d'atomes d e silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.
De manière plus spécifique, par "polymère non élastomére" on désigne un polymère ayant une recouvrance instantanée $R_j$ < à 50% et une recouvrance retardée $R_{2h}$ < 70% après avoir subi un allongement de 30%. De préférence, $R_i$ est < à 30 %, et $R_{2h}$ < 50.

Plus précisément, le caractère non élastomérique du polymère est déterminé selon le protocole suivant :

> On prépare un film de polymère par coulage d'une solution du polymère dans une matrice téflonnée puis séchage pendant 7 jours dans une ambiance contrôlée à 23±5°C et 50±10 % d'humidité relative.
> On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($I_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($I_0$)
- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i) / \varepsilon_{max}) \times 100$$

Pour déterminer la recouvrance retardée, on mesure après 2 heures le taux d'allongement résiduel de l'éprouvette en pourcentage ($\varepsilon_{2h}$), 2 heures après retour à la contrainte charge nulle.

La recouvrance retardée en % ($R_{2h}$) est donnée par la formule ci-après:

$$R_{2h} = (\varepsilon_{max} - \varepsilon_{2h}) / \varepsilon_{max}) \times 100$$

A titre purement indicatif, un polymère selon un mode de réalisation de l'invention possède de préférence une recouvrance instantanée $R_i$ de 10% et une recouvrance retardée $R_{2h}$ de 30%.

Le polymère selon l'invention comprend au moins une première séquence (ou bloc) et au moins une deuxième séquence (ou bloc) incompatibles l'une avec l'autre et ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence, ledit polymère ayant un indice de polydispersité I supérieur à 2,5.

On précise que dans ce qui précède et ce qui suit les termes "première" et "deuxième" séquences ne conditionnent nullement l'ordre desdites séquences (ou blocs) dans la structure du polymère.

L'indice de polydispersité I d polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère selon l'invention est de préférence inférieure ou égale à 300 000, elle va par exemple de 35 000 à 200 000, et mieux de 45 000 à 150 000.

La masse moyenne en nombre (Mn) du polymère selon l'invention est de préférence inférieure ou égale à 70 000, elle va par exemple de 10 000 à 60 000, et mieux de 12 000 à 50 000.

De préférence, l'indice de polydispersité du polymère selon l'invention est supérieur à 2,5, par exemple allant de 2,5 à 8, et mieux supérieur ou égal à 2,8 et notamment, allant de 2,8 à 6.

Chaque séquence ou bloc du polymère selon l'invention est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par " essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Selon l'invention, les première et deuxième séquences ont des températures de transition vitreuse différentes.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i) ,$$

étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

En particulier, la première séquence peut être choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et la deuxième séquence choisie dans une catégorie a), b) ou c) différente de la première séquence.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

a) Séquence ayant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, notamment allant de 50 °C à 100 °C, et mieux supérieure ou égale à 60°C, allant par exemple de 60°C à 120°C .

La séquence ayant une Tg supérieure ou égale à 40°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin, .

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$

dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,

- les acrylates de formule $CH_2 = CH\text{-}COOR_2$

dans laquelle $R_2$ représente un groupe cycloalkyle en $C_4$ à $C_{12}$ tel que un groupe isobornyle ou un groupe tertio butyle,

- les (méth)acrylamides de formule :

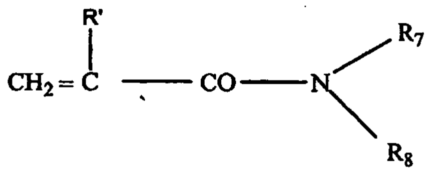

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide,

- et leurs mélanges.

Des monomères principaux particulièrement préférés sont le méthacrylate de méthyle, le (méth)acrylate d'isobutyle, le (méth)acrylate d'isobornyle et leurs mélanges.

b) Séquence ayant une Tg inférieure ou égale à 20°C

La séquence ayant une Tg inférieure ou égale à 20°C a par exemple une Tg allant de -100 à 20°C, de préférence

inférieure ou égale à 15°C, notamment allant de -80°C à 15°C et m ieux inférieure ou égale à 10°C, par exemple allant de - 100 °C à 0 °C, notamment allant de -50°C à 0°C.

La séquence ayant une Tg inférieure ou égale à 20°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de— 80°C à 15°C et mieux inférieur à 10°C, par exemple allant de —50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-}CO\text{-}O\text{-}CH = CH_2$
  où $R_5$ représente un groupe alkyle en $C_4$ à $C_{12}$ linéaire ou ramifié ;

- les éthers d'alcool vinylique et d'alcool en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

<u>c) Séquence ayant une Tg comprise entre 20 et 40°C</u>

La séquence qui a une Tg comprise entre 20 et 40°C peut être un homopolymère ou un copolymère.

Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).

Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.

Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,
  lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.
De préférence, la proportion de la deuxième séquence ayant une Tg inférieure ou égale à 20°C va de 10 à 85% en poids du polymère, mieux de 20 à 70% et encore mieux de 20 à 50%.
Chacune des séquences peut néanmoins contenir en proportion minoritaire au moins un monomère constitutif de l'autre séquence.
Ainsi la première séquence peut contenir au moins un monomère constitutif de la deuxième séquence et inversement.
Chacune des première et/ou deuxième séquence, peu(ven)t comprendre, outre les monomères indiqués ci-dessus, un ou plusieurs autres monomères appelés monomères additionnels, différents des monomères principaux cités précédemment.
La nature et la quantité de ce ou ces monomères additionnels sont choisies de manière à ce que la séquence dans laquelle ils se trouvent ait la température de transition vitreuse désirée.
Ce monomère additionnel est par exemple choisi parmi,:

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy ) silane,

- et leurs mélanges.

Des monomères additionnels particulièrement préférés sont l'acide acrylique, l'acide méthacrylique, le méthacrylate de trifluoroéthyle et leurs mélanges.

Selon un mode préféré de réalisation, le polymère selon l'invention est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

De p référence, chacune d es p remière et deuxième séquences comprend au moins un monomère choisi parmi les esters d'acide (méth)acrylique, et éventuellement au moins un monomère choisi parmi l'acide (méth)acrylique, et leurs mélanges.

Avantageusement, chacune des première et deuxième séquences est issue en totalité d'au moins un monomère choisi parmi l'acide acrylique, les esters d'acide (méth)acrylique, et éventuellement d'au moins un monomère choisi parmi l'acide (méth)acrylique, et leurs mélanges

De préférence, le polymère selon l'invention est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène. de styrène ou de dérivés du styrène comme par exemple le méthylstyrène, le chlorostyrène ou le chlorométhylstyrène.

Le polymère selon l'invention peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' ( allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

<u>Premier mode de réalisation</u>

Selon un premier mode de réalisation, le polymère selon l'invention comprend au moins une (notamment une) première séquence ayant une Tg supérieure ou égale à 40°C, telle que décrite plus haut au a) et au moins une (notamment une) deuxième séquence ayant une Tg inférieure ou égale à 20°C, telle que décrite plus haut au b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C va de 20 à 90% en poids du polymère, mieux de 30 à 80% et encore mieux de 50 à 70%. De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Selon une troisième variante, le polymère selon l'invention peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon une quatrième variante, le polymère selon l'invention peut comprendre :

- une première séquence de Tg supérieure ou égale à 40°C, par exemple allant de 85 à 115°C, qui est un copolymère acrylate d'isobornyle/méthacrylate de méthyle,
- une deuxième séquence de Tg inférieure ou égale à 20°C, par exemple allant de -85 à - 55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère selon l'invention peut comprendre :

- une première séquence de Tg supérieure ou égale à 40°C, par exemple allant de 95 à 125°C, qui est un copolymère acrylate d'isobornyle / méthacrylate d'isobornyle,
- une deuxième séquence de Tg inférieure ou égale à 20°C, par exemple allant de -85 à - 55°C, qui est un homopolymère d'acrylate d'éthyl-2 hexyle et
- une séquence intermédiaire qui est un copolymère statistique acrylate d'isobornyle/méthacrylate d'isobornyle/ acrylate d'éthyl-2 hexyle.

Selon une sixième variante, le polymère selon l'invention peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/ acrylate d'isobutyle.

Selon une septième variante, le polymère selon l'invention peut comprendre :

- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à - 5°C, qui est un homopolymère d'acrylate d'isobutyle et
- une séquence intermédiaire qui est un copolymère statistique acrylate d'isobornyle/méthacrylate d'isobornyle/acrylate d'isobutyle.

Selon une huitième variante, le polymère selon l'invention peut comprendre :

- une première séquence de Tg supérieure ou égale à 40°C, par exemple allant de 60 à 90°C, qui est un copolymère acrylate d'isobornyle/méthacrylate d'isobutyle,

- une deuxième séquence de Tg inférieure ou égale à 20°C, par exemple allant de -35 à - 5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Les exemples qui suivent illustrent de manière non limitative des polymères correspondant à ce premier mode de réalisation.
Les quantités sont exprimées en gramme.

**Exemple 1 : Préparation d'un polymère de poly(méthacrylate de méthyle/acide acryliquer/acrylate de méthyle)**

100 g d'acétate de butyle sont introduits dans un réacteur de 1 litre, puis on augmente la température de façon à passer de la température ambiante (25°C) à 90°C en 1 heure. On ajoute ensuite, à 90°C et en 1 heure, 180 g de méthacrylate de méthyle, 30 g d'acide acrylique, 40 g d'acétate de butyle, 70 g d'isopropanol et 1,8 g de 2.5- Bis (2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
On obtient un polymère comprenant une première séquence ou bloc poly (méthacrylate de méthyle/acide acrylique) ayant une Tg de 100°C une deuxième séquence ou bloc polyacrylate de méthyle ayant une Tg de 10°C et une séquence intermédiaire qui est un polymère statistique méthacrylate de méthyle/acide acrylique/polyacrylate de méthyle.
Ce polymère présente une masse moyenne en poids de 52000 et une masse moyenne en nombre de 18000, soit un indice de polydispersité I de 2,89

**Exemple 2 : Préparation d'un polymère de poly (méthacrylate de méthyle/acide acrylique/méthacrylate de méthyle)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 150g de méthacrylate de méthyle, 30 g d'acide acrylique, 30 g d'acrylate de méthyle, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
On obtient un polymère comprenant une première séquence ou bloc poly (acide acrylique/acrylate de méthyle) ayant une Tg de 80°C, une deuxième séquence polyacrylate de méthyle ayant une Tg de 10°C et une séquence intermédiaire qui est un polymère statistique acide acrylique/acrylate de méthyle/polyacrylate de méthyle.
Ce polymère présente une masse moyenne en poids de 50 000 et une masse moyenne en nombre de 17 000, soit un indice de polydispersité I de 2,95.

**Exemple 3 : Préparation d'un polymère de poly(acide acrylique/acrylate de méthyle/polyacrylate de méthyle/méthacrylate de trifluoroéthyle)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 120 g de méthacrylate de méthyle, 30 g d'acide acrylique, 60 g de méthacrylate de trifluoroéthyle, 40 g d'acétate de butyle, 70 g d'isopropanol et 1,8 g de 2.5- Bis(2-éthylhexanoylperoxy)-2.5-diméthylhexane (Trigonox 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate

de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.

On obtient un polymère comprenant une première séquence ou bloc poly (acide acrylique/méthacrylate de méthyle/méthacrylate de trifluoroéthyle) ayant une Tg de 85°C, une deuxième séquence polyacrylate de méthyle ayant une Tg de 10°C et une séquence intermédiaire qui est un polymère statistique acide acrylique/acrylate de méthyle/polyacrylate de méthyle/méthacrylate de trifluoroéthyle.

Ce polymère présente une masse moyenne en poids de 53 000 et une masse moyenne en nombre de 17 500, soit un indice de polydispersité I de 3,03.

**Exemple 4 : Préparation d'un polymère de poly(acrylate d'isobornyle/méthacrylate d'isobutyle/acrylate d'éthyl-2 hexyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 120 g d'acrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate d'isobutyle) ayant une Tg de 80°C, une deuxième séquence polyacrylate d'éthyl-2 hexyle ayant une Tg de - 70°C et une séquence intermédiaire qui est un polymère statistique acrylate d'isobornyle/méthacrylate d'isobutyle/acrylate d'éthyl-2 hexyle.

Ce polymère présente une masse moyenne en poids de 77 000 et une masse moyenne en nombre de 19 000, soit un indice de polydispersité I de 4,05.

**Exemple 5 : Préparation d'un polymère de poly(acrylate d'isobornyle/méthacrylate de méthyle/acrylate d'éthyl-2 hexyle**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 150 g d'acrylate d'isobornyle, 60 g de méthacrylate de méthyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et. 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate de méthyle) ayant une Tg de 100°C, une deuxième séquence polyacrylate d'éthyl-2 hexyle ayant une Tg de - 70°C et une séquence intermédiaire qui est un polymère statistique acrylate d'isobornyle/méthacrylate de méthyle/acrylate d'éthyl-2 hexyle.

Ce polymère présente une masse moyenne en poids de 76 500 et une masse moyenne en nombre de 22 000, soit un indice de polydispersité I de 3,48.

**Exemple 6 : Préparation d'un polymère de poly(acrylate d'isobornyle/méthacrylate de méthyle/acrylate d'éthyl-2 hexyle.**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 90 g d'acrylate d'isobornyle, 60 g de méthacrylate de méthyle, 50 g d'isododécane et 1,5 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 1 heure, 150 g d'acrylate d'éthyl-2 hexyle, 150 g

d'isododécane et 1,5 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate de méthyle) ayant une Tg de 100°C , une deuxième séquence polyacrylate d'éthyl-2 hexyle ayant une Tg de -70°C et une séquence intermédiaire qui est un polymère statistique acrylate d'isobornyle/méthacrylate de méthyle/acrylate d'éthyl-2 hexyle.

Ce polymère présente une masse moyenne en poids de 76 500 et une masse moyenne en nombre de 22 000, soit un indice de polydispersité I de 3,48.

**Exemple 7 : Préparation d'un polymère de poly(acrylate d'isobornyle/méthacrylate d'isobornyle lacrylate d'éthyl-2 hexyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate d'isobornyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.

Ce polymère présente une masse moyenne en poids de 103 900 et une masse moyenne en nombre de 21 300, soit un indice de polydispersité I de 4.89.

**Exemple 8 : Préparation d'un polymère de poly(methacrylate d'isobornyle/methacrylate d'isobutyle /acrylate d'isobutyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 120 g de méthacrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox®141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(methacrylate d'isobornyle/méthacrylate d'isobutyle) ayant une Tg de 95°C, une deuxième séquence polyacrylate d'isobutyle ayant une Tg de - 20°C et une séquence intermédiaire qui est un polymère statistique méthacrylate d'isobornyle/méthacrylate d'isobutyle/acrylate d'isobutyle.

Ce polymère présente une masse moyenne en poids de 100 700 et une masse moyenne en nombre de 20 800, soit un indice de polydispersité I de 4.85.

**Exemple 9 : Préparation d'un polymère de poly(acrylate d'isobornyle/méthacrylate d'isobornyle /acrylate d'isobutyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate d'isobornyle) ayant une Tg de 110°C, une deuxième séquence polyacrylate d'isobutyle ayant une Tg de - 20°C et une séquence intermédiaire qui est un polymère statistique acrylate d'isobornyle/méthacrylate d'isobornyle/acrylate d'isobutyle.

Ce polymère présente une masse moyenne en poids de 151 000 et une masse moyenne en nombre de 41 200, soit un indice de polydispersité I de 3.66.

**Exemple 10 : Préparation d'un polymère de poly(acrylate d'isobornyle/methacrylate d'isobutyle /acrylate d'isobutyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 120 g de acrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate d'isobutyle) ayant une Tg de 75°C, une deuxième séquence polyacrylate d'isobutyle ayant une Tg de - 20°C et une séquence intermédiaire qui est un polymère statistique acrylate d'isobornyle/méthacrylate d'isobutyle/acrylate d'isobutyle.

Ce polymère présente une masse moyenne en poids de 144 200 et une masse moyenne en nombre de 49 300, soit un indice de polydispersité I de 2,93.

Second mode de réalisation

Selon un second mode de réalisation, le polymère selon l'invention comprend au moins une ( notamment u ne) p remière s équence a yant une température de transition vitreuse (Tg) comprise entre 20 et 40°C, conforme aux séquences décrites au c) et au moins une (notamment une) deuxième séquence ayant une température de transition vitreuse inférieure ou égale à 20°C, telle que décrite plus haut au b) ou une température de transition vitreuse supérieure ou égale à 40°C, telle que décrite au a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

. Avantageusement, la deuxième séquence ayant une Tg inférieure ou égale à 20°C ou ayant une Tg supérieure ou égale à 40°C est un homopolymère.

Ainsi, selon première variante de ce second mode de réalisation, le polymère selon l'invention peut comprendre :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et

au moins un monomère acrylate de méthyle.

Selon seconde variante de ce second mode de réalisation, le polymère selon l'invention peut comprendre :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à -35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère selon l'invention peut comprendre :

- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg isupérieure ou égale à 40°C, par exemple allant de 85 à 115°C, qui est un homopolymère d'acrylate d'isobornyle et
- une séquence intermédiaire qui est un copolymère statistique acrylate d'isobornyle /acrylate de méthyle/acide acrylique.

A titre illustratif mais non limitatif les polymères correspondant à ce second mode de réalisation peuvent être réalisés comme suit.

**Exemple 11 : Préparation d'un polymère de poly(méthacrylate de butyle/acrylate de butyle )**

100 g d'acétate de butyle sont introduits dans un réacteur de 1 litre, puis on augmente la température de façon à passer de la température ambiante 25°C à 90°C en 1 heure.
On ajoute ensuite, à 90°C et en 1 heure, 210 g de méthacrylate de butyle , 110 g d'acétate de butyle et 1,8 g de .2.5-Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 1 heure, 90 g d'acrylate de butyle et 90 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
On obtient un polymère comprenant une première séquence ou bloc polyméthacrylate de butyle ayant une Tg de 25°C, une deuxième séquence ou bloc polyacrylate de butyle ayant une Tg de - 50°C° et une séquence intermédiaire qui est un polymère statistique méthacrylate de butyle /acrylate de butyle.
Ce polymère présente une masse moyenne en poids de 57560 et une masse moyenne en nombre de 19025, soit un indice de polydispersité I de 3.03

**Exemple 12 : Préparation d'un polymère de poly(méthacrylate de méthyle/acrylate de méthyle/acide acrylique)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 50,4 g de méthacrylate de méthyle, 21 g d'acide acrylique, 138,6 g d'acrylate de méthyle, 40 g d'acétate de butyle, 70 g d'isopropanol et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g de méthacrylate de méthyle, 70 g d'acétate de butyle, 20 g d'isopropanol et 1,2 g de 2.5-Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle ) ayant une Tg de 100°C et une séquence intermédiaire qui est un polymère statistique méthacrylate de méthyle/acide acrylique/ polyacrylate de méthyle.

**Exemple 13 : Préparation d'un polymère de poly(acrylate d'isobornyle/méthacrylate d'isobutyle/acrylate d'éthyl-2 hexyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 50,4 g de méthacrylate d'isobutyle, 54.6 g d'acrylate d'éthyle-2 hexyle, 110g d'isododécane et 1,8 g de 2.5-Bis(2-éthylhexanoylperoxy)-2.5-diméthylhexane (Trigonox 141 d'Akzo Nobel).
Le mélange est maintenu 1 h 30 à 90°C.
On introduit ensuite au mélange précédent, toujours à 90°C et en 1 heure, 90 g d'acrylate d'éthyle-2 hexyle, 90 g d'isododécane et 1,2 g de 2.5-Bis(2-éthylhexanoylperoxy)-2.5-diméthylhexane.
Le mélange est maintenu 3 heures à 90°C, puis dilué puis l'ensemble est refroidi.
On obtient une solution à 50% de matière active en polymère dans l'isododécane.
Le polymère obtenu comprend une première séquence ou bloc poly(acrylate d'isobornyle/méthacrylate d'isobutyle/ acrylate d'éthyl-2 hexyle) ayant une Tg de 35°C, une deuxième séquence polyacrylate d'éthyl-2 hexyle ayant une Tg de -50°C et une séquence intermédiaire qui est un polymère statistique acrylate d'isobornyle/méthacrylate d'isobutyle/acrylate d'éthyl-2 hexyle.

**Exemple 14 : Préparation d'un polymère de poly(acrylate d'isobornyle/méthacrylate d'isobutyle/acrylate d'éthyl-2 hexyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 54 g d'acrylate d'isobornyle, 75,6 g de méthacrylate d'isobutyle, 50,4 g d'acrylate d'éthyle-2 hexyle , 110g d'isododécane et 1,8 g de 2.5-Bis(2-éthylhexanoylperoxy)-2.5-diméthylhexane (Trigonox 141 d'Akzo Nobel).
Le mélange est maintenu 1 h 30 à 90°C.
On introduit ensuite au mélange précédent, toujours à 90°C et en 1 heure, 120 g d'acrylate d'éthyle-2 hexyle, 90 g d'isododécane et 1,2 g de 2.5-Bis(2-éthylhexanoylperoxy)-2.5-diméthylhexane.
Le mélange est maintenu 3 heures à 90°C, puis dilué puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobutyle/ acrylate d'éthyl-2 hexyle) ayant une Tg de 25°C, une deuxième séquence polyacrylate d'éthyl-2 hexyle ayant une Tg de -50°C et une séquence intermédiaire qui est un polymère statistique acrylate d'isobornyle/méthacrylate d'isobutyle/acrylate d'éthyl-2 hexyle.

**Exemple 15 : Préparation d'un polymère poly(acrylate d'isobornyle/acide acrylique/acrylatede méthyle**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 210 g d'acrylate d'isobornyle, 110 g d'isododécane et 1,8 g de 2.5-Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 1 heure, 18 g d'acrylate d'isobornyle, 71,1 g d'acrylate de méthyle, 0,9 g d'acide acrylique, 90 g d'isododécane et 1,2 g de 2.5-Bis(2-éthylhexanoylperoxy)-2.5-diméthylhexane.
Le mélange est maintenu 3 heures à 90°C, puis dilué puis l'ensemble est refroidi.
On obtient une solution à 50% de matière active en polymère dans l'isododécane.
On obtient un polymère comprenant une première séquence ou bloc poly(acrylate d'isobornyle/acrylate de méthyle/acide acrylique ) ayant une Tg de 25°C, une deuxième séquence polyacrylate d'isobornyle ayant une Tg de 100°C et une séquence intermédiaire qui est un polymère statistique (acrylate d'isobornyle/acrylate de méthyle/acide acrylique)
On a préparé le polymère suivant :

**Exemple 16 : Préparation d'un polymère de poly(méthacrylate de méthyle/acrylate de méthyle/acide acrylique)**

210 g d'acétate d'éthyle sont introduits dans un réacteur de 1 litre, puis on augmente la température de façon à passer de la température ambiante (25°C) à 78°C en 1 heure.
On ajoute ensuite, à 78°C et en 1 heure, 54 g de méthacrylate de méthyle, 21 g d'acide acrylique, 135 g d'acrylate de

méthyle et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 78°C et en 1 heure, 90 g de méthacrylate de méthyle, 90 g d'acétate d'éthyle et 1,2 g de 2.5-Bis(2-éthylhexanoylperoxy)-2.5-diméthylhexane.
Le mélange est maintenu 3 heures à 78°C, puis dilué par 150 g d'acétate d'éthyle puis l'ensemble est refroidi.
On obtient une solution à 40% de matière active en polymère dans l'acétate d'éthyle.

Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle ) ayant une Tg de 100°C et une séquence intermédiaire qui est un polymère statistique méthacrylate de méthyle/acide acrylique/ polyacrylate de méthyle.

Ce polymère présente une masse moyenne en poids de 141 000 et une masse moyenne en nombre de 50 000, soit un indice de polydispersité I de 2,82

L'invention concerne également des compositions cosmétiques comprenant au moins un polymère de structure spécifique, tel qu'il a été décrit ci-dessus.

Généralement, ces compositions contiennent de 0,1 à 60% en poids en matière active (ou matière sèche) du polymère selon l'invention, de préférence de 0,5 à 50 % en poids, et de préférence encore de 1 à 40% en poids.

Ces compositions cosmétiques, selon l'invention, comprennent, outre lesdits polymères, un milieu physiologiquement acceptable, c'est-à-dire un milieu compatible avec les matières kératiniques, comme la peau, les cheveux, les cils, les sourcils et les ongles.

Ledit milieu, physiologiquement acceptable, comprend généralement un solvant approprié, physiologiquement acceptable.

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.
L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

La composition peut comprendre, outre le polymère séquence décrit précédemment selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.
Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les c yclométhicones, les diméthicones, comportant éventuellement un groupement phényle, comme les phényl triméthicones, les phényltriméthylsiloxydiphényl siloxanes, les diphénylméthyldiméthyltrisiloxanes,

les diphényl diméthicones, les phényl diméthicones, les polyméthylphényl siloxanes ; leurs mélanges.
Ces huiles peuvent être présentes en une teneur allant de 0,01 à 90 %, et mieux de 0,1 à 85 % en poids, par rapport au poids total de la composition.

Par corps gras pâteux, on entend un produit visqueux contenant une fraction liquide et une fraction solide. Par « corps gras pâteux » au sens de l'invention, on entend des corps gras ayant un point de fusion allant de 20 à 55 °C, de préférence 25 à 45 °C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé.
Les valeurs de point de fusion correspondent, selon l'invention, au pic de fusion mesurée par la méthode "Differential Scanning Calorimetry" avec une montée en température de 5 ou 10 °C/min.

On peut utiliser un ou plusieurs corps gras pâteux. De préférence, ces corps gras sont des composés hydrocarbonés (contenant principalement des atomes de carbone et d'hydrogène et éventuellement des groupements ester), éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly (12-hydroxystéarique) et leurs mélanges. Comme triglycérides d'origine végétale, on peut utiliser les dérivés d'huile de ricin hydrogénée, tels que le "THIXINR" de Rheox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le ou les corps gras pâteux peuvent être présents à raison de 0,5 à 60% en poids, par rapport au poids total de la composition, de préférence à raison de 2-45% en poids et encore plus préférentiellement à raison de 5-30% en poids, dans la composition.

La composition selon l'invention peut également comprendre un ou plusieurs solvants organiques, cosmétiquement acceptables (tolérance, toxicologie et toucher acceptables).

Ces solvants peuvent être généralement présents en une teneur allant de 0 à 90 %, de préférence de 0,1 à 90%, de préférence encore de 10 à 90% en poids, par rapport au poids total de la composition, et mieux de 30 à 90 %.

Comme solvants utilisables dans la composition de l'invention, on peut citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.
En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
La cire peut également présenter une dureté allant de 0,05 MPa à 15 MPa, et de préférence allant de 6 MPa à 15 MPa .
La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant

à la vitesse de mesure de 0,1 mm/s, et pénétrant dans la cire à une profondeur de pénétration de 0,3 mm.
Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.
Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 % en poids.
Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre en outre une ou plusieurs charges, notamment en une teneur allant de 0 ,01 % à 5 0 % en poids, p ar rapport a u poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine, et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique,

tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.
Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les produits pour les lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara pour cheveux).

Lorsque la composition selon l'invention est une composition de maquillage pour le teint, elle comprend de préférence un polymère conforme au polymère du premier mode de réalisation décrit précédemment, notamment comprenant une séquence ayant une Tg allant de 50 °C à 100 °C et une séquence ayant une Tg allant de - 100 °C à 0 °C, et plus particulièrement un polymère choisi parmi le polymère de la troisième variante et le polymère de la huitième variante du premier mode de réalisation, décrits précédemment.

La composition selon l'invention peut également être un produit de soin de la peau du corps et du visage, notamment un produit solaire ou de coloration de la peau (tel qu'un autobronzant).

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage.

Les exemples qui suivent illustrent de manière non limitative les compositions comprenant le polymère selon l'invention.
Les quantités sont exprimées en gramme.

**<u>Exemple 17 : Vernis à ongles</u>**

On a préparé un vernis à ongles ayant la composition suivante

| | |
|---|---|
| Polymère de l'exemple 1 | 23,8 g en MA |
| Acétate de Butyle | 24,99 g |
| Isopropanol | 10,71 g |
| Hexylène Glycol | 2,5 g |
| DC RED 7 Lake | 1 g |
| Hectorite modifiée par du chlorure de stéaryl di méthyl benzyl ammonium (Bentone® 27V d'Elementis) | 1,3 g |
| Acétate d'éthyle | qsp 100 g |

Après application sur les ongles, ce vernis a été jugé comme présentant de très bonnes propriétés de tenue et de résistance aux chocs.

**<u>Exemple 18 : Composition de mascara</u>**

On a préparé un mascara ayant la composition suivante :

| | |
|---|---|
| Cire d'abeille | 8 g |

(suite)

| | |
|---|---|
| Cire de paraffine | 3 g |
| Cire de carnauba | 6 g |
| Hectorite modifiée par du chlorure de di-stéaryl di-méthyl benzyl ammonium (Bentone® 38V d'Elementis) | 5,3 g |
| Carbonate de propylène | 1,7 g |
| Charge | 1 g |
| pigments | 5 g |
| Polymère de l'exemple 4 | 12 g en MA |
| Isododécane | qsp 100 |

La tenue du film de mascara, après application sur les cils, a été jugée comme très satisfaisante.

**Exemple 19 : Composition de mascara**

On a préparé un mascara ayant la composition suivante :

| | |
|---|---|
| Cire d'abeille | 8 g |
| Cire de paraffine | 3 g |
| Cire de carnauba | 6 g |
| Hectorite modifiée par du chlorure de di-stéaryl di-méthyl benzyl ammonium (Bentone® 38V d'Elementis) | 5,3 g |
| Carbonate de propylène | 1,7 g |
| Charge | 1 g |
| pigments | 5 g |
| Polymère de l'exemple 7 | 12 g en MA |
| Isododécane | qsp 100 |

La tenue du film de mascara, après application sur les cils, a été jugée comme très satisfaisante.

**Exemple 20 : Stick de rouge à lèvres**

La composition de rouge à lèvres suivante est préparée :

| | |
|---|---|
| Cire de polyéthylène | 15 % |
| Polymère de l'exemple 5 | 10 % en MA |
| Polyisobutène hydrogéné (Parléam de Nippon Oil Fats) | 26 % |
| Isododécane | qsp 100 |
| Pigments | 8.6 % |

Le film de composition obtenu après application sur les lèvres présente de bonnes propriétés de tenue.

**Exemple 21 : Fond de teint E/H**

On prépare une composition de fond de teint comprenant les composés suivants :

| | | |
|---|---|---|
| Phase A | Cetyl Dimethicone copolyol (ABIL EM 90 de la société GOLDSCHMIDT) | 3 g |
| | Succinate d'isostéaryl diglycéryle (IMWITOR 780K de la société CONDEA) | 0,6 g |
| | Isododécane | 18,5 g |
| | Mélange de pigments (oxydes de fer et oxydes de titane hydrophobes) | 10 g |
| | Polymère de l'exemple 5 | 8,7 g en MA |
| | Poudre de polyamide (NYLON-12 de Dupont de Nemours) | 8 g |
| | Parfum | 0,5 g |

(suite)

| | | |
|---|---|---|
| Phase B | Eau | qsp 100 |
| | Sulfate de magnésium | 0,7 g |
| | Conservateur (Methylparaben) | 0,2 g |
| Phase C | Eau | 2 g |
| | Conservateur (Diazolinyl urée) | 0,25 g |

**Exemple 22 : Vernis à ongles**

| | |
|---|---|
| Polymère de l'exemple 12 | 23,8 g en MA |
| Acétate de Butyle | 24,99 g |
| Isopropanol | 10,71 g |
| Hexylène Glycol | 2,5 g |
| DC RED 7 Lake | 1 g |
| Hectorite modifiée par du chlorure de stéaryl di méthyl benzyl ammonium (Bentone® 27V d'Elementis) | 1,3 g |
| Acétate d'éthyle | qsp 100 g |

**Exemple 23 : Composition de mascara**

| | |
|---|---|
| Cire d'abeille | 8 g |
| Cire de paraffine | 3 g |
| Cire de carnauba | 6 g |
| Hectorite modifiée par du chlorure de di-stéaryl di-méthyl benzyl ammonium (Bentone® 38V d'Elementis) | 5,3 g |
| Carbonate de propylène | 1,7 g |
| Charge | 1 g |
| pigments | 5 g |
| Polymère de l'exemple 14 | 12 g en MA |
| Isododécane | qsp 100 |

**Exemple 24 : Stick de rouge à lèvres**

| | |
|---|---|
| Cire de polyéthylène | 15 % |
| Polymère de l'exemple 13 | 10 % en MA |
| Polyisobutène hydrogéné (Parléam de Nippon Oil Fats) | 26 % |
| Isododécane | qsp 100 |
| Pigments | 8.6 % |

**Exemple 25 : Fond de teint E/H**

| | | |
|---|---|---|
| Phase A | Cetyl Dimethicone copolyol (ABIL EM 90 de la société GOLDSCHMIDT) | 3 g |
| | Succinate d'isostéaryl diglycéryle (IMWITOR 780K de la société CONDEA) | 0,6 g |
| | Isododécane | 18,5 g |
| | Mélange de pigments (oxydes de fer et oxydes de titane hydrophobes) | 10 g |

(suite)

| | | |
|---|---|---|
| | Polymère de l'exemple 15 | 8,7 g en MA |
| | Poudre de polyamide (NYLON-12 de Dupont de Nemours) | 8 g |
| | Parfum | 0,5 g |
| Phase B | Eau | qsp 100 |
| | Sulfate de magnésium | 0,7 g |
| | Conservateur (Methylparaben) | 0,2 g |
| Phase C | Eau | 2 g |
| | Conservateur (Diazolinyl urée) | 0,25 g |

**<u>Exemple 26 : Fond de teint E/H</u>**

On a préparé une composition de fond de teint comprenant les composés suivants :

| | | |
|---|---|---|
| Phase A | Mélange de polydiméthylsiloxane oxyéthyléné oxypropyléné et de cyclopentasiloxane (85/15) (ABIL EM 97 de la société GOLDSCHMIDT) | 1,8 g |
| | Succinate d'isostéaryl diglycéryle (IMWITOR 780K de la société CONDEA) | 0,6 g |
| | Cyclopentasiloxane | 6 g |
| | Isododécane qsp | 100 g |
| | Mélange de pigments (oxydes de fer et oxydes de titane hydrophobes) | 10 g |
| | Polymère de l'exemple 4 | 2,1 g en MA |
| | Poudre de polyamide (NYLON-12 de Dupont de Nemours) | 8 g |
| Phase | B Eau | 41,4 g |
| | Sulfate de magnésium | 0,7 g |
| | Conservateur | 0,3 g |

**<u>Exemple 27 : Fond de teint E/H</u>**

On a préparé une composition de fond de teint comprenant les composés suivants :

| | | |
|---|---|---|
| Phase A | Mélange de polydiméthylsiloxane oxyéthyléné oxypropyléné et de cyclopentasiloxane (85/15) (ABIL EM 97 de la société GOLDSCHMIDT) | 1,8 g |
| | Succinate d'isostéaryl diglycéryle (IMWITOR 780K de la société CONDEA) | 0,6 g |
| | Cyclopentasiloxane | 6 g |
| | Isododécane qsp | 100 g |
| | Mélange de pigments (oxydes de fer et oxydes de titane hydrophobes) | 10 g |
| | Polymère de l'exemple 10 | 2,1 g en MA |
| | Poudre de polyamide (NYLON-12 de Dupont de Nemours) | 8 g |
| Phase | B Eau | 41,4 g |
| | Sulfate de magnésium | 0,7 g |
| | Conservateur | 0,3 g |

**Revendications**

**1.** Polymère comprenant au moins une première séquence et au moins une deuxième séquence incompatibles l'une avec l'autre et ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence; ledit polymère ayant un indice de polydispersité I supérieur à 2,5; ladite première séquence étant choisie parmi :

a) une séquence ayant une Tg supérieure ou égale à 40°C,
b) une séquence ayant une Tg inférieure ou égale à 20°C,
c) une séquence ayant une Tg comprise entre 20 et 40°C, et

ladite deuxième séquence étant choisie dans une catégorie a), b) ou c) différente de la première séquence; ledit segment intermédiaire étant un polymère statistique.

2. Polymère selon la revendication 1, dans lequel la première séquence a une température de transition vitreuse (Tg) supérieure ou égale à 40°C et la deuxième séquence a une température de transition vitreuse inférieure ou égale à 20°C.

3. Polymère selon la revendication 1, dans lequel la première séquence ayant une température de transition vitreuse (Tg) comprise entre 20 et 40°C et la deuxième séquence ayant une température de transition vitreuse inférieure ou égale à 20°C ou une température de transition vitreuse supérieure ou égale à 40°C:

4. Polymère selon l'une des revendications précédentes, **caractérisé en ce que** la séquence ayant une Tg supérieure ou égale à 40°C est issue en totalité ou en partie de un ou plusieurs monomères, qui sont tels que l'homopolymère préparé à partir de ces monomères a une températures de transition vitreuse supérieure ou égale à 40°C.

5. Polymère selon la revendication 4, **caractérisé en ce que** les monomères dont l'homopolymère correspondant a une température de transition vitreuse supérieure ou égale à 40°C sont choisis parmi les monomères suivants :

   - les méthacrylates de formule $CH_2 = C(CH_3)\text{-}COOR_1$
   dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$.
   - les acrylates de formule $CH_2 = CH\text{-}COOR_2$
   dans laquelle $R_2$ représente un groupe cycloalkyle en $C_4$ à $C_{12}$ tel que l'acrylate d'isobornyle ou un groupe tertio butyle,
   - les (méth)acrylamides de formule $CH_2=C(R')\text{-}CO\text{-}NR_7R_8$
   où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.
   - et leurs mélanges.

6. Polymère selon l'une des revendications 4 à 5, **caractérisé en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

7. Polymère selon l'une des revendications précédentes, **caractérisé en ce que** la séquence ayant une Tg inférieure ou égale à 20°C est issue en totalité ou en partie de un ou plusieurs monomères, qui sont tel(s) que l'homopolymère préparé à partir de ces monomères a une température de transition vitreuse inférieure ou égale à 20°C.

8. Polymère selon la revendication 7, **caractérisé en ce que** les monomères dont l'homopolymère correspondant a une température de transition vitreuse inférieure ou égale à 20°C sont choisis parmi les monomères suivants :

   - les acrylates de formule $CH_2 = CHCOOR_3$,
   $R_3$ représentant un groupe alkyle non substitué en $C_1$ à $C_{12}$ linéaire ou ramifié, à l'exception du groupe tertiobutyle, dans lequel se trouve(nt) éventuellement intercalé(s) un ou plusieurs hétèroatomes choisis parmi O, N, S ;
   - les méthacrylates de formule $CH_2 = C(CH_3)\text{-}COOR_4$,
   $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi O, N et S;
   - les esters de vinyle de formule $R_5\text{-}CO\text{-}O\text{-}CH = CH_2$
   où $R_5$ représente un groupe alkyle en $C_4$ à $C_{12}$ linéaire ou ramifié ;
   - les éthers d'alcool vinylique et d'alcool en $C_4$ à $C_{12}$;
   - les N-alkyl en $C_4$ à $C_{12}$ acrylamides, tels que le N-octylacrylamide,
   et leurs mélanges.

9. Polymère selon l'une des revendications 7 à 8, **caractérisé en ce que** les monomères dont l'homopolymère correspondant a une température de transition vitreuse inférieure ou égale à 20°C sont choisis parmi les acrylates d'alkyle dont la chaine alkyle comprend de 1 à 10 atomes de carbone, à l'exception du groupe tertiobutyle

**10.** Polymère selon l'une des revendications précédentes, **caractérisé en ce que** la séquence ayant une Tg comprise entre 20 et 40°C est issue en totalitè ou en partie de un ou de plusieurs monomères, qui sont tel(s) que l'homopolymère préparés à partir de ces monomères a une température de transition vitreuse comprise entre 20 et 40°C.

**11.** Polymère selon l'une des revendications 1 à 9, **caractérisé en ce que** la séquence ayant une Tg comprise entre 20 et 40°C est issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**12.** Polymère selon l'une des revendications précédentes, **caractérisé en ce que** la séquence ayant une Tg comprise entre 20 et 40°C est issue en totalité ou en partie de monomères choisis parmi te méthacrylate de méthyle, l'acrylate et le méthacrylate d'isobornyle, le méthacrylate de trifuoroéthyle, l'acrylate de butyle, l'acrylate d'éthyl-2 hexyle et leurs mélanges.

**13.** Polymère selon la revendication 2, **caractérisé en ce que** la proportion de la séquence ayant une Tg supérieure ou égale à 40°C, va de 20 à 90% en poids du polymère, mieux de 30 à 80% et encore mieux de 50 à 70%.

**14.** Polymère selon la revendication 2, **caractérisé en ce que** la proportion de la séquence ayant une Tg inférieure ou égale à 20°C va de 5 à 75% en poids du polymère, mieux de 15 à 50% et encore mieux de 25 à 45%.

**15.** Polymère selon la revendication 3, **caractérisé en ce que** la proportion de la séquence ayant une Tg comprise entre 20 et 40°C va de 10 à 85% en poids du polymère, mieux de 30 à 80% et encore mieux de 50 à 70%.

**16.** Polymère selon la revendication 3, **caractérisé en ce que** la proportion de la séquence ayant une Tg supérieure ou égale à 40°C va de 10 à 85%, de préférence de 20 à 70% et mieux de 30 à 70% en poids du polymère.

**17.** Polymère selon la revendication 3, **caractérisé en ce que** la proportion de la séquence ayant une température de transition vitreuse inférieure ou égale à 20°C va de 10 à 85% en poids du polymère, mieux de 20 à 70% et encore mieux de 20 à 50%.

**18.** Polymère selon l'une des revendications précédentes, **caractérisé en ce que** la première séquence et/ou la deuxième séquence comprend au moins un monomère additionnel.

**19.** Polymère selon la revendication 18, **caractérisé en ce que** le monomère additionnel est choisi parmi les monomères hydrophiles, les monomères à insaturation éthylénique comprenant un ou plusieurs atomes de silicium et leurs mélanges.

**20.** Polymère selon l'une des revendications 18 à 19, **caractérisé en ce que** le monomère additionnel est choisi parmi :

- les monomères à insaturation(s) éthylénique(s) comprenant au moins une fonction acide carboxylique ou sulfonique
- les méthacrylates de formule $CH_2 = C(CH_3)\text{-}COOR_6$
dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl. Br, I, F) ;
- les acrylates de formule $CH_7 = CHCOOR_{10}$,
$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène

**21.** Polymère selon l'une des revendications 18 à 20, **caractérisé en ce que** le ou les monomères additionnels sont

choisis parmi l'acide acrylique, l'acide méthacrylique, le méthacrylate de trifluoroéthyle et leurs mélanges.

**22.** Polymère selon l'une des revendications 18 à 21, **caractérisé en ce que** le ou les monomères additionnel(s) représente(nt) de 1 à 30% en poids du poids total des première et/ou deuxième séquences.

**23.** Polymère selon l'une des revendications précédentes, **caractérisé en ce que** chacune des première et deuxième séquence comprend au moins un monomère choisi parmi les esters d'acide (meth)acrylique, et éventuellement au moins un monomère choisi parmi l'acide (meth)acrylique et leurs mélanges.

**24.** Polymère selon l'une des revendications précédentes, **caractérisé en ce que** chacune des première et deuxième séquence est issue en totalité d'au moins un monomère choisi parmi l'acide acrylique, les esters d'acide (méth) acrylique, et éventuellement d'au moins un monomère choisi parmi l'acide (méth)acrylique, et leurs mélanges

**25.** Polymère selon l'une des revendications précédentes, **caractérisé en ce** l'écart entre les températures de transition vitreuse (Tg) des première et deuxième séquences est supérieur à 10°C, mieux, supérieur à 20°C, de préférence supérieure à 30°C et mieux supérieure à 40°C.

**26.** Polymére selon l'une des revendications précédentes, **caractérisé en ce que** la séquence intermédiaire a une température de transition vitreuse comprise entre les températures de transition vitreuse des première et deuxième séquences.

**27.** Polymère selon l'une des revendications précédentes, **caractérisé en ce qu'**il a un indice de polydispersité supérieur ou égal à 2,8.

**28.** Polymère selon l'une des revendications précédentes, **caractérisé en ce qu'**il a un indice de polydispersité compris entre 2,8 et 6.

**29.** Polymère selon l'une des revendications précédentes, dont la masse moyenne en poids (Mw) est inférieure ou égale à 300 000.

**30.** Polymère selon l'une des revendications précédentes, dont la masse moyenne en poids (Mw) va de 35 000 à 200 000. et mieux de 45 000 à 150 000.

**31.** Polymère selon l'une des revendications précédentes, dont la masse moyenne en nombre (Mn) est inférieure ou égale à 70 000.

**32.** Polymère selon l'une des revendications précédentes, dont la masse moyenne en nombre (Mn) va de 10 000 à 60 000, et mieux de 12 000 à 50 000.

**33.** Polymère selon l'une des revendications précédentes, **caractérisé en ce qu'**il n'est pas soluble dans l'eau ou dans un mélange d'eau et de monoalcools inférieurs linéaires ou ramifiés ayant de 2 à 5 atomes de carbone, sans modification de pH, à une teneur en matière active d'au moins 1% en poids, à température ambiante (25°C).

**34.** Polymère selon l'une des revendications précédentes, **caractérisé en ce qu'**il n'est pas un élastomère.

**35.** Polymère selon l'une des revendications précédentes, **caractérisé en ce qu'**il est un polymère éthylénique séquencé linéaire filmogène.

**36.** Procédé de préparation d'un polymère selon l'une des revendications précédentes, comprenant les étapes suivantes :

- une partie du solvant de polymérisation est introduite dans un réacteur adapté et chauffée jusqu'à atteindre la température adéquate pour la polymérisation,
- une fois cette température atteinte, les monomères constitutifs de la première séquence sont introduits en présence d'amorceur de polymérisation,
- au bout d'un temps T correspondant à un taux de conversion maximum de 90%, les monomères constitutifs de la deuxième séquence et l'autre partie du solvant de polymérisation sont introduits,
- on laisse réagir le mélange pendant un temps T' au bout duquel le mélange est refroidi,

- on obtient le polymère en solution dans le solvant de polymérisation.

**37.** Procédé selon la revendication 36, **caractérisé en ce que** la température de polymérisation est de 60-120°C.

**38.** Composition cosmétique **caractérisée en ce qu'**elle comprend un polymère selon l'une quelconque des revendications 1 à 35.

**39.** Composition cosmétique selon la revendication 38, **caractérisée en ce qu'**elle comprend de 0,1 à 60 % en poids en matière active de polymère, de préférence de 5 % à 50% en poids, et de préférence encore de 10 à 40 % en poids.

**40.** Composition selon l'une des revendications 38 à 39, **caractérisée en ce qu'**elle comprend un milieu physiologiquement acceptable.

**41.** Composition selon l'une des revendications 38 à 40, **caractérisée en ce que** le milieu physiologiquement acceptable comprend un milieu hydrophile comprenant de l'eau et les mélanges d'eau et de solvant(s) organique(s) hydrophile(s), tels que les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols.

**42.** Composition selon l'une des revendications 38 à 41, **caractérisée en ce qu'**elle comprend, en outre, une phase grasse composée de corps gras liquides ou solides à température ambiante, d'origine animale, végétale, minérale ou synthétique.

**43.** Composition selon l'une des revendications 38 à 42, **caractérisée en ce qu'**elle comprend, en outre, un ou plusieurs solvants organiques cosmétiquement acceptables.

**44.** Composition selon l'une des revendications 38 à 43**, caractérisée en ce qu'**elle comprend, en outre, un ou plusieurs agents auxiliaires de filmification choisis parmi les agents plastifiants et les agents de coalescence.

**45.** Composition selon l'une des revendications 38 à 44, **caractérisée en ce qu'**elle comprend, en outre, une ou des matières colorantes choisies parmi les colorants hydrosolubles et les matières colorantes pulvérulentes, tels que les pigments, les nacres et les paillettes.

**46.** Composition selon l'une des revendications 38 à 45, **caractérisée en ce qu'**elle comprend, en outre, des charges.

**47.** Composition selon l'une des revendications 38 à 46, **caractérisée en ce qu'**elle comprend, en outre, un ou plusieurs ingrédient(s) choisis parmi les vitamines, les épaississants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents anti-pelliculaires les agents propulseurs et leurs mélanges.

**48.** Composition selon l'une des revendications 38 à 47, **caractérisée en ce qu'**elle se présente sous forme de suspension, de dispersion, de solution, de gel, d'émulsion, notamment émulsion huile-dans-eau (H/E) ou eau-dans-huile (E/H), ou multiplie (E/H/E ou polyol/H/F ou H/E/H), sous forme de crème, de pâte, de mousse, de dispersion de vésicules notamment de lipides ioniques ou non, de lotion biphase ou multiphase, de poudre, de pâte, notamment de pâte souple ou de pâte anhydre.

**49.** Composition selon l'une des revendications 38 à 48, **caractérisée en ce qu'**il s'agit d'une composition de maquillage ou de soin des matières kératiniques.

**50.** Composition selon l'une des revendications 38 à 49, **caractérisée en ce qu'**il s'agit d'un produit capillaire.

**51.** Composition selon l'une des revendications 38 à 49, **caractérisée en ce qu'**il s'agit d'un vernis à ongles.

**52.** Composition selon l'une des revendications 38 à 49, **caractérisée en ce qu'**il s'agit d'un produit de maquillage des lèvres.

**53.** Composition selon l'une des revendications 38 à 49, **caractérisée en ce qu'**il s'agit d'un produit de maquillage des yeux.

**54.** Composition selon l'une des revendications 38 à 49, **caractérisée en ce qu'**il s'agit d'un produit de maquillage du teint.

**55.** Composition selon la revendication 54, **caractérisée en ce que** le polymère séquencé comprend une séquence ayant une Tg allant de 50"C à 100°C et une séquence ayant une Tg allant de -100°C à 0°C.

**56.** Composition selon la revendication 54, **caractérisée en ce que** le polymère séquencé est choisi parmi :

(i) un polymère séquencé comprenant :

- une première séquence de Tg supérieure ou égale à 40°C, par exemple allant de 85 à 115°C, qui est un copolymère acrylate d'isobornyle/méthacrylate d'isobutyle.
- une deuxième séquence de Tg inférieure ou égale à 20°C, par exemple allant de -85 à
- 55°C, qui est un homopolymère d'acrylate d'éthyl-2 hexyte, et
- une séquence intermédiaire qui est un copolymère statistique acrylate d'isobornyle/méthacrylate d'isobutyle/acrylate d'éthyl-2 hexyle.

(ii) un polymère séquencé comprenant :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique acrylate d'isobornyle/méthacrylate d'isobutyle/acrylate d'isobutyle.

**57.** Composition selon l'une des revendications 38 à 56, **caractérisée en ce qu'**elle comprend un polymère filmogène additionnel.

**58.** Composition selon l'une des revendications 38 à 57, **caractérisée en ce qu'**elle comprend un corps gras solide choisi parmi les cires, les corps gras pâteux, les gommes, et leurs mélanges.

**59.** Procédé cosmétique de maquillage ou de soin des matières kératiniques comprenant l'application sur les matières kératiniques d'une composition cosmétique selon l'une des revendications 38 à 58.

**60.** Utilisation d'un polymère selon l'une quelconque des revendications 1 à 37, dans une composition cosmétique, comme agent pour améliorer la tenue de ladite composition.

**61.** Utilisation d'un polymère selon l'une quelconque des revendications 1 à 3, dans une composition présentant des propriétés de tenue améliorées.

## Claims

**1.** Polymer comprising at least one first block and at least one second block which are incompatible with one another and that have different glass transition temperatures ($T_g$) said first and second blocks being connected to one another via an intermediate segment comprising at least one constituent monomer of the first block and at least one constituent monomer of the second block; said polymer having a polydispersity index I of greater than 2.5; said first block being chosen from:

a) a block having a $T_g$ of greater than or equal to 40°C;
b) a block having a $T_g$ of less than or equal to 20°C;
c) a block having a $T_g$ of between 20°C, and 40°C; and said second block being chosen from a category a), b) or c) different from the first block;

said intermediate segment being a random polymer.

**2.** Polymer according to Claim 1, in which the first block has a glass transition temperature ($T_g$) of greater than or equal

to 40°C and the second block has a glass transition temperature of less than or equal to 20°C.

3. Polymer according to Claim 1, in which the first block has a glass transition temperature ($T_g$) of between 20°C and 40°C and the second block has a glass transition temperature of less than or equal to 20°C or a glass transition temperature of greater than or equal to 40°C.

4. Polymer according to one of the preceding claims, **characterized in that** the block having a $T_g$ of greater than or equal to 40°C results, in full or in part, from one or more monomers which are such that the homopolymer prepared from these monomers has a glass transition temperature of greater than or equal to 40°C.

5. Polymer according to Claim 4, **characterized in that** the monomers for which the corresponding homopolymer has a glass transition temperature of greater than or equal to 40°C are chosen from the following monomers:

   methacrylates of formula $CH_2=C(CH_3)-COOR_1$,
   in which $R_1$ represents a linear or branched unsubstituted alkyl group comprising from 1 to 4 carbon atoms, such as a methyl, ethyl, propyl or isobutyl groups, or $R_1$ represents a $C_4$ to $C_{12}$ cycloalkyl group;
   - acrylates of formula $CH_2=CH-COOR_2$,
   in which $R_2$ represents a $C_4$ to $C_{12}$ cycloalkyl group, such as isobornyl acrylate, or a tert-butyl group;
   - (meth)acrylamides of formula $CH_2=C(R')-CO-NR_7R_8$,
   where $R_7$ and $R_8$, which are identical or different, each represent a hydrogen atom or a linear or branched alkyl group having 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl;
   - and mixtures thereof.

6. Polymer according to either of Claims 4 and 5, **characterized in that** the monomers for which the corresponding homopolymer has a glass transition temperature of greater than or equal to 40°C are chosen from methyl methacrylate, isobutyl methacrylate, isobornyl (meth)acrylate, and mixtures thereof.

7. Polymer according to one of the preceding claims, **characterized in that** the block having a $T_g$ of less than or equal to 20°C results, in full or in part, from one or more monomers which are such that the homopolymer prepared from these monomers has a glass transition temperature of less than or equal to 20°C.

8. Polymer according to Claim 7, **characterized in that** the monomers for which the corresponding homopolymer has a glass transition temperature of less than or equal to 20°C are chosen from the following monomers:

   - acrylates of formula $CH_2=CHCOOR_3$,
   $R_3$ representing a linear or branched unsubstituted $C_1$ to $C_{12}$ alkyl group with the exception of the tert-butyl group, in which occur(s), optionally intercalated, one or more heteroatoms chosen from O, N and S;
   - methacrylates of formula $CH_2=C(CH_3)-COOR_4$,
   $R_4$ representing a linear or branched unsubstituted $C_6$ to $C_{12}$ alkyl group, in which occur(s), optionally intercalated, one or more heteroatoms chosen from O, N and S;
   - vinyl esters of formula $R_5-CO-O-CH=CH_2$,
   where $R_5$ represents a linear or branched $C_4$ to $C_{12}$ alkyl group;
   - $C_4$ to $C_{12}$ alcohol and vinyl alcohol ethers;
   - N-($C_4$ to $C_{12}$ alkyl) acrylamides, such as N-octylacrylamide,
   - and mixtures thereof.

9. Polymer according to either of Claims 7 and 8, **characterized in that** the monomers for which the corresponding homopolymer has a glass transition temperature of less than or equal to 20°C are chosen from alkyl acrylates for which the alkyl chain comprises from 1 to 10 carbon atoms, with the exception of the tert-butyl group.

10. Polymer according to one of the preceding claims, **characterized in that** the block having a $T_g$ of between 20°C and 40°C results, in full or in part, from one or more monomers which are such that the homopolymer prepared from these monomers has a glass transition temperature of between 20°C and 40°C.

11. Polymer according to one of Claims 1 to 9, **characterized in that** the block having a $T_g$ of between 20°C and 40°C results, in full or in part, from monomers which are such that the corresponding homopolymer has a $T_g$ of greater than or equal to 40°C and from monomers which are such that the corresponding homopolymer has a $T_g$ of less

than or equal to 20°C.

**12.** Polymer according to one of the preceding claims, **characterized in that** the block having a $T_g$ of between 20°C, and 40°C results, in full or in part, from monomers chosen from methyl methacrylate, isobornyl acrylate, isobornyl methacrylate, trifluoroethyl methacrylate, butyl acrylate, 2-ethylhexyl acrylate, and mixtures thereof.

**13.** Polymer according to Claim 2, **characterized in that** the proportion of the block having a $T_g$ of greater than or equal to 40°C ranges from 20 to 90% by weight, better still from 30 to 80% by weight and even better still from 50 to 70% by weight of the polymer.

**14.** Polymer according to Claim 2, **characterized in that** the proportion of the block having a $T_g$ of less than or equal to 20°C ranges from 5 to 75% by weight, better still from 15 to 50% by weight and even better still from 25 to 45% by weight of the polymer.

**15.** Polymer according to Claim 3, **characterized in that** the proportion of the block having a Tg of between 20°C and 40°C ranges from 10 to 85% by weight, better still from 30 to 80% by weight and even better still from 50 to 70% by weight of the polymer.

**16.** Polymer according to Claim 3, **characterized in that** the proportion of the block having a $T_g$ of greater than or equal to 40°C ranges from 10 to 85% by weight, preferably from 20 to 70% by weight and better still from 30 to 70% by weight of the polymer.

**17.** Polymer according to Claim 3, **characterized in that** the proportion of the block having a glass transition temperature of less than or equal to 20°C ranges from 10 to 85% by weight, better still from 20 to 70% by weight and even better still from 20 to 50% by weight of the polymer.

**18.** Polymer according to one of the preceding claims, **characterized in that** the first block and/or the second block comprises at least one additional monomer.

**19.** Polymer according to Claim 18, **characterized in that** the additional monomer is chosen from hydrophilic monomers, monomers having ethylenically unsaturated groups comprising one or more silicon atoms, and mixtures thereof.

**20.** Polymer according to either of Claims 18 and 19, **characterized in that** the additional monomer is chosen from:

- monomers having ethylenically unsaturated group(s) comprising at least one carboxylic or sulfonic acid functional group;
- methacrylates of formula $CH_2=C(CH_3)-COOR_6$,
in which $R_6$ represents a linear or branched alkyl group comprising from 1 to 4 carbon atoms, such as a methyl, ethyl, propyl or isobutyl group, said alkyl group being substituted by one or more substituents chosen from hydroxyl groups (such as 2-hydroxypropyl methacrylate, 2-hydroxyethyl methacrylate) and halogen atoms (Cl, Br, I, F), such as trifluoroethyl methacrylate;
- methacrylates of formula $CH_2=C(CH_3)-COOR_9$,
$R_9$ representing a linear or branched $C_6$ to $C_{12}$ alkyl group, in which occur(s), optionally intercalated, one or more heteroatoms chosen from O, N and S, said alkyl group being substituted by one or more substituents chosen from hydroxyl groups and halogen atoms (Cl, Br, I, F) ;
- acrylates of formula $CH_2=CHCOOR_{10}$,
$R_{10}$ representing a linear or branched $C_1$ to $C_{12}$ alkyl group substituted by one or more substituents chosen from hydroxyl groups and halogen atoms (Cl, Br, I and F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit from 5 to 30 times, for example methoxy-POE, or $R_8$ represents a polyoxyethylene group comprising from 5 to 30 ethylene oxide units.

**21.** Polymer according to one of Claims 18 to 20, **characterized in that** the additional monomer(s) is/are chosen from acrylic acid, methacrylic acid, trifluoroethyl methacrylate and mixtures thereof.

**22.** Polymer according to one of Claims 18 to 21, **characterized in that** the additional monomer(s) represent(s) from 1 to 30% by weight of the total weight of the first and/or second blocks.

**23.** Polymer according to one of the preceding claims, **characterized in that** each of the first and second blocks comprises at least one monomer chosen from (meth)acrylic acid esters, and optionally at least one monomer chosen from (meth)acrylic acid and mixtures thereof.

**24.** Polymer according to one of the preceding claims, **characterized in that** each of the first and second blocks results, in full, from at least one monomer chosen from acrylic acid, (meth)acrylic acid esters, and optionally at least one monomer chosen from (meth)acrylic acid and mixtures thereof.

**25.** Polymer according to one of the preceding claims, **characterized in that** the difference between the glass transition temperatures ($T_g$) of the first and second blocks is greater than 10°C, better still greater than 20°C, preferably greater than 30°C, and better still greater than 40°C.

**26.** Polymer according to one of the preceding claims, **characterized in that** the intermediate block has a glass transition temperature between the glass transition temperatures of the first and second blocks.

**27.** Polymer according to one of the preceding claims, **characterized in that** it has a polydispersity index of greater than or equal to 2.8.

**28.** Polymer according to one of the preceding claims, **characterized in that** it has a polydispersity index of between 2.8 and 6.

**29.** Polymer according to one of the preceding claims, the weight-average molecular weight ($M_w$) of which is less than or equal to 300 000.

**30.** Polymer according to one of the preceding claims, the weight-average molecular weight ($M_w$) of which ranges from 35 000 to 200 000 and better still from 45 000 to 150 000.

**31.** Polymer according to one of the preceding claims, the number-average molecular weight ($M_n$) of which is less than or equal to 70 000.

**32.** Polymer according to one of the preceding claims, the number-average molecular weight ($M_n$) of which ranges from 10 000 to 60 000 and better still from 12 000 to 50 000.

**33.** Polymer according to one of the preceding claims, **characterized in that** it is insoluble in water or in a mixture of water and of linear or branched lower monoalcohols having 2 to 5 carbon atoms, without pH modification, at an active material content of at least 1% by weight, at ambient temperature (25°C).

**34.** Polymer according to one of the preceding claims, **characterized in that** it is not an elastomer.

**35.** Polymer according to one of the preceding claims, **characterized in that** it is a film-forming, linear, block ethylenic polymer.

**36.** Process for the preparation of a polymer according to one of the preceding claims, which comprises the following stages:

- a portion of the polymerization solvent is introduced into a suitable reactor and is heated until the temperature appropriate for the polymerization is reached;
- once this temperature is reached, the constituent monomers of the first block are introduced in the presence of polymerization initiator;
- at the end of a time T corresponding to a maximum degree of conversion of 90%, the constituent monomers of the second block and the other portion of the polymerization solvent are introduced;
- the mixture is reacted for a time T', at the end of which the mixture is cooled;
- the polymer is obtained in solution in the polymerization solvent.

**37.** Process according to Claim 36, **characterized in that** the polymerization temperature is 60-120°C.

**38.** Cosmetic composition, **characterized in that** it comprises a polymer according to one of Claims 1 to 35.

**39.** Cosmetic composition according to Claim 38, **characterized in that** it comprises from 0.1 to 60% by weight, preferably from 5 to 50% by weight and more preferably from 10 to 40% by weight of polymer active material.

**40.** Composition according to either of Claims 38 and 39, **characterized in that** it comprises a physiologically acceptable medium.

**41.** Composition according to one of Claims 38 to 40, **characterized in that** the physiologically acceptable medium comprises a hydrophilic medium comprising water and mixtures of water and of hydrophilic organic solvent(s), such as alcohols and in particular linear or branched lower monoalcohols having from 2 to 5 carbon atoms, such as ethanol, isopropanol or n-propanol, and polyols, such as glycerol, diglycerol, propylene glycol, sorbitol, pentylene glycol and polyethylene glycols.

**42.** Composition according to one of Claims 38 to 41, **characterized in that** it additionally comprises a fatty phase composed of fatty substances which are liquid or solid at ambient temperature and which are of animal, plant, mineral or synthetic origin.

**43.** Composition according to one of Claims 38 to 42, **characterized in that** it additionally comprises one or more cosmetically acceptable organic solvents.

**44.** Composition according to one of Claims 38 to 43, **characterized in that** it additionally comprises one or more auxiliary film-forming agents chosen from plasticizing agents and coalescing agents.

**45.** Composition according to one of Claims 38 to 44, **characterized in that** it additionally comprises one or more dyestuffs chosen from water-soluble dyes and pulverulent dyestuffs, such as pigments, pearlescent agents and glitter.

**46.** Composition according to one of Claims 38 to 45, **characterized in that** it additionally comprises fillers.

**47.** Composition according to one of Claims 38 to 46, **characterized in that** it additionally comprises one or more ingredient(s) chosen from vitamins, thickeners, trace elements, emollients, sequestering agents, fragrances, basifying or acidifying agents, preservatives, sunscreens, surfactants, antioxidants, agents for combating hair loss, antidandruff agents, propellants and mixtures thereof.

**48.** Composition according to one of Claims 38 to 47, **characterized in that** it is in the form of a suspension, dispersion, solution, gel, emulsion, in particular oil-in-water (O/W) or water-in-oil (W/O) or multiple (W/O/W or polyol/O/W or O/W/O) emulsion, in the form of a cream, foam, dispersion of vesicles, in particular of ionic or nonionic lipids, two-phase or multiphase lotion, powder, paste, in particular soft paste or anhydrous paste.

**49.** Composition according to one of Claims 38 to 48, **characterized in that** it is a composition for making up or caring for keratin materials.

**50.** Composition according to one of Claims 38 to 49, **characterized in that** it is a hair product.

**51.** Composition according to one of Claims 38 to 49, **characterized in that** it is a nail varnish.

**52.** Composition according to one of Claims 38 to 49, **characterized in that** it is a product for making up the lips.

**53.** Composition according to one of Claims 38 to 49, **characterized in that** it is a product for making up the eyes.

**54.** Composition according to one of Claims 38 to 49, **characterized in that** it is a product for making up the complexion.

**55.** Composition according to Claim 54, **characterized in that** the block polymer comprises a block having a $T_g$ ranging from 50°C to 100°C and a block having a $T_g$ ranging from -100°C to 0°C.

**56.** Composition according to Claim 54, **characterized in that** the block polymer is chosen from:

(i) a block polymer comprising:

- a first block having a $T_g$ of greater than or equal to 40°C, for example ranging from 85°C to 115°C, which

is an isobornyl acrylate/isobutyl methacrylate copolymer;
- a second block having a $T_g$ of less than or equal to 20°C, for example ranging from -85°C to -55°C, which is a 2-ethylhexyl acrylate homopolymer; and
- an intermediate block which is a random isobornyl acrylate/isobutyl methacrylate/2-ethylhexyl acrylate copolymer;

(ii) a block polymer comprising:

- a first block having a $T_g$ of greater than or equal to 40°C, for example ranging from 60°C to 90°C, which is an isobornyl acrylate/isobutyl methacrylate copolymer;
- a second block having a $T_g$ of less than or equal to 20°C, for example ranging from -35°C to -5°C, which is an isobutyl acrylate homopolymer; and
- an intermediate block which is a random isobornyl acrylate/isobutyl methacrylate/isobutyl acrylate copolymer.

**57.** Composition according to one of Claims 38 to 56, **characterized in that** it comprises an additional film-forming polymer.

**58.** Composition according to one of Claims 38 to 57, **characterized in that** it comprises a solid fatty substance chosen from waxes, pasty fatty substances, gums and mixtures thereof.

**59.** Cosmetic method for making up or caring for keratin materials, which comprises the application, to the keratin materials, of a cosmetic composition according to one of Claims 38 to 58.

**60.** Use of a polymer according to any one of Claims 1 to 37, in a cosmetic composition as an agent for improving the hold of said composition.

**61.** Use of a polymer according to any one of Claims 1 to 3, in a composition exhibiting improved hold properties.

**Patentansprüche**

**1.** Polymer, das zumindest eine erste Sequenz und zumindest eine zweite Sequenz umfasst, die nicht miteinander kompatibel sind und die unterschiedliche Glasübergangstemperaturen (Tg) aufweisen, wobei die erste Sequenz und die zweite Sequenz über ein Zwischensegment aneinander gebunden sind, das mindestens ein die erste Sequenz aufbauendes Monomer und mindestens ein die zweite Sequenz aufbauendes Monomer umfasst, wobei das Polymer einen Polydispersitätsindex größer 2,5 aufweist; wobei die erste Sequenz ausgewählt ist unter:

a) einer Sequenz mit einer Tg von größer oder gleich 40 °C;
b) einer Sequenz mit einer Tg von kleiner oder gleich 20 °C,;
c) einer Sequenz mit einer Tg zwischen 20 und 40 °C;

und die zweite Sequenz aus einer Gruppe a), b) oder c) ausgewählt ist, die von der ersten Sequenz verschieden ist; und wobei es sich bei dem Zwischensegment um ein statistisches Polymer handelt.

**2.** Polymer nach Anspruch 1, wobei die erste Sequenz eine Glasübergangstemperatur (Tg) von größer oder gleich 40°C aufweist und die zweite Sequenz eine Glasübergangstemperatur von kleiner oder gleich 20 °C aufweist.

**3.** Polymer nach Anspruch 1, wobei die erste Sequenz eine Glasübergangstemperatur (Tg) zwischen 20 und 40 °C aufweist und die zweite Sequenz eine Glasübergangstemperatur von kleiner oder gleich 20 °C oder eine Glasübergangstemperatur von größer oder gleich 40 °C aufweist.

**4.** Polymer nach einem der vorhergehenden Ansprüche, **dadurch gekennzeichnet, dass** die Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

**5.** Polymer nach Anspruch 4, **dadurch gekennzeichnet, dass** die Monomere, deren entsprechendes Homopolymer

eine Glasübergangstemperatur von größer oder gleich 40 °C aufweist, unter den folgenden Monomeren ausgewählt sind:

- Methacrylaten der Formel $CH_2=C(CH_3)-COOR_1$, worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen ist,
- Acrylaten der Formel $CH_2=CH-COOR_2$, worin $R_2$ eine Cycloalkylgruppe mit 4 bis 12 Kohlenstoffatomen, wie Isobornylacrylat, oder tert-Butyl bedeutet,
- (Meth)acrylamiden der Formel $CH_2=C(R')-CO-NR_7R_8$, worin $R_7$ und $R_8$, die gleich oder verschieden sind, jeweils ein Wasserstoffatom oder eine lineare oder verzweigte Alkylgruppe mit 1 bis 12 Kohlenstoffatomen, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
- und deren Gemischen.

6. Polymer nach einem der Ansprüche 4 bis 5, **dadurch gekennzeichnet, dass** die Monomere, deren entsprechendes Homopolymer eine Glasübergangstemperatur von größer oder gleich 40 °C aufweist unter Methylmethacrylat, Isobutylmethacrylat und Isobomyl(meth)acrylat und deren Gemischen ausgewählt sind.

7. Polymer nach einem der vorhergehenden Ansprüche, **dadurch gekennzeichnet, dass** die Sequenz mit einer Tg von kleiner oder gleich 20 °C ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

8. Polymer nach Anspruch 7, **dadurch gekennzeichnet, dass** die Monomere, deren entsprechendes Homopolymer eine Glasübergangstemperatur von kleiner oder gleich 20 °C, aufweist, unter den folgenden Monomeren ausgewählt sind:

- Acrylaten der Formel $CH_2=CHCOOR_3$, worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2=C(CH_3)-COOR_4$, worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5-CO-O-CH=CH_2$, worin $R_5$ eine lineare oder verzweigte Alkylgruppe mit 4 bis 12 Kohlenstoffatomen bedeutet,
- Ethern eines Vinylakohols und eines $C_{4-12}$-Alkohols,
- N-Alkyl($C_{4-12}$)acrylamiden, wie N-Octylacrylamid,
- und deren Gemischen.

9. Polymer nach einem der Ansprüche 7 bis 8, **dadurch gekennzeichnet, dass** die Monomere, deren entsprechendes Homopolymer eine Glasübergangstemperatur von kleiner oder gleich 20 °C aufweist, mit Ausnahme der tert-Butylgruppe unter den Alkylacrylaten ausgewählt sind, deren Alkylgruppe 1 bis 10 Kohlenstoffatome aufweist.

10. Polymer nach einem der vorhergehenden Ansprüche, **dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweiset.

11. Polymer nach einem der Ansprüche 1 bis 9, **dadurch gekennzeichnet, dass** die Sequenz mit einer Tg zwischen 20 und 40°C ganz oder teilweise von einem oder mehreren Monomeren, die so sind, dass das entsprechende Homopolymer eine Glasübergangstemperatur von größer oder gleich 40 °C aufweist, und von Monomeren abgeleitet ist, die so sind, dass das entsprechende Homopolymer eine Glasübergangstemperatur von kleiner oder gleich 20 °C aufweist.

12. Polymer nach einem der vorhergehenden Ansprüche, **dadurch gekennzeichnet, dass** die Sequenz mit einer Tg zwischen 20 und 40°C ganz oder teilweise von Monomeren abgeleitet ist, die unter Methylmethacrylat, Isobornylacrylat, Isobornylmethacrylat, Trifluorethylmethacrylat, Butylacrylat und 2-Ethylhexylacrylat und deren Gemischen ausgewählt sind.

**13.** Polymer nach Anspruch 2, **dadurch gekennzeichnet, dass** der Mengenanteil der Sequenz mit einer Tg von größer oder gleich 40 °C im Bereich von 20 bis 90 Gew.-% des Polymers, besser im Bereich von 30 bis 80 Gew.-% und noch besser im Bereich von 50 bis 70 Gew.-% liegt.

**14.** Polymer nach Anspruch 2, **dadurch gekennzeichnet, dass** der Mengenanteil der Sequenz mit einer Tg von kleiner oder gleich 20 °C im Bereich von 5 bis 75 Gew.-% des Polymers, besser im Bereich von 15 bis 50 Gew.-% und noch besser im Bereich von 25 bis 45 Gew.-% liegt.

**15.** Polymer nach Anspruch 3, **dadurch gekennzeichnet, dass** der Mengenanteil der Sequenz mit einer Tg zwischen 20 und 40°C im Bereich von 10 bis 85 Gew.-% des Polymers, besser im Bereich von 30 bis 80 Gew.-% und noch besser im Bereich von 50 bis 70 Gew.-% liegt.

**16.** Polymer nach Anspruch 3, **dadurch gekennzeichnet, dass** der Mengenanteil der Sequenz mit einer Tg von größer oder gleich 40 °C im Bereich von 10 bis 85 Gew.-% des Polymers, besser im Bereich von 20 bis 70 Ges.-% und noch besser im Bereich von 30 bis 70 Gew.-% liegt.

**17.** Polymer nach Anspruch 3, **dadurch gekennzeichnet, dass** der Mengenanteil der Sequenz mit einer Glasübergangstemperatur von kleiner oder gleich 20 °C im Bereich von 10 bis 85 Gew.-% des Polymers, besser im Bereich von 20 bis 70 Gew.-% und noch besser im Bereich von 20 bis 50 Gew.-% liegt.

**18.** Polymer nach einem der vorhergehenden Ansprüche, **dadurch gekennzeichnet, dass** die erste Sequenz und/ oder die zweite Sequenz mindestens ein zusätzliches Monomer aufweisen.

**19.** Polymer nach Anspruch 18, **dadurch gekennzeichnet, dass** das zusätzliche Monomer unter den hydrophilen Monomeren, den Monomeren mit ethylenisch ungesättigter Bindung, die ein oder mehrere Siliciumatome enthalten, und deren Gemischen ausgewählt ist.

**20.** Polymer nach einem der Ansprüche 18 bis 19, **dadurch gekennzeichnet, dass** das zusätzliche Monomer ausgewählt ist unter:

- Monomeren mit einer oder mehreren ethylenisch ungesättigten Bindungen, die mindestens eine Carbonsäurefunktion oder Sulfonsäurefunktion enthalten,
- Methacrylaten der Formel $CH_2=C(CH_3)-COOR_6$, worin $R_6$ eine lineare oder verzweigte Alkylgruppe mit 1 bis 4 Kohlenstoffatomen bedeutet, wie Methyl, Ethyl, Propyl oder Isobutyl, wobei die Alkylgruppe mit einem oder mehreren Substituenten substituiert ist, die unter den Hydroxygruppen (z.B. 2-Hydroxypropylmethacrylat und 2-Hydroxyethylmethacrylat) und Halogenatomen (Cl, Br, I oder F), wie Trifluorethylmethacrylat, ausgewählt sind,
- Methacrylaten der Formel $CH_2=C(CH_3)-COOR_9$, worin $R_9$ eine lineare oder verzweigte Alkylgruppe mit 6 bis 12 Kohlenstoffatomen bedeutet, in die optional ein oder mehrere Heteroatome, die unter O, N und S ausgewählt sind, eingebaut ist (sind), wobei die Alkylgruppe mit einem oder mehreren Substituenten substituiert ist, die unter den Hydroxygruppen und den Halogenatomen (Cl, Br, I und F) ausgewählt sind,
- Acrylaten der Formel $CH_2=CHCOOR_{10}$, worin $R_{10}$ eine lineare oder verzweigte Alkylgruppe mit 1 bis 12 Kohlenstoffatomen bedeutet, die mit einem oder mehreren Substituenten substituiert ist, die unter den Hydroxygruppen und den Halogenatomen (Cl, Br, I und F) ausgewählt sind, wie 2-Hydroxypropylacrylat und 2-Hydroxyethylacrylat, oder $R_{10}$ eine Gruppe $C_{1-12}$-Alkyl-O-POE (Polyoxyethylen) bedeutet, wobei die Oxyethyleneinheit 5 bis 30 Mal wiederholt wird, beispielsweise Methoxy-POE, oder $R_{10}$ eine Polyoxyethylengruppe bedeutet, die 5 bis 30 Ethylenoxideinheiten aufweist.

**21.** Polymer nach einem der Ansprüche 18 bis 20, **dadurch gekennzeichnet, dass** das oder die zusätzlichen Monomere unter Acrylsäure, Methacrylsäure und Trifluorethylmethacrylat und deren Gemischen ausgewählt sind.

**22.** Polymer nach einem der Ansprüche 18 bis 21, **dadurch gekennzeichnet, dass** das oder die zusätzliche(n) Monomer (e) 1 bis 30 Gew.-% des Gesamtgewichts der ersten und/oder zweiten Sequenz ausmachen.

**23.** Polymer nach einem der vorhergehenden Ansprüche, **dadurch gekennzeichnet, dass** die erste und zweite Sequenz jede zumindest ein zusätzliches Monomer enthält, das unter (Meth)acrylsäureestem und optional mindestens einem ergänzenden Monomer, wie (Meth)acrylsäure, und deren Gemischen ausgewählt ist.

**24.** Polymer nach einem der vorhergehenden Ansprüche, **dadurch gekennzeichnet, dass** die erste und zweite Sequenz jede vollständig von zumindest einem Monomer abgeleitet ist, das unter Acrylsäure, (Meth)acrylsäureestern und optional mindestens einem Monomer, das unter (Meth)acrylsäure ausgewählt ist, und deren Gemischen ausgewählt ist.

**25.** Polymer nach einem der vorhergehenden Ansprüche, **dadurch gekennzeichnet, dass** die Differenz der Glasübergangstemperaturen (Tg) der ersten und der zweiten Sequenz größer als 10 °C, besser größer als 20 °C, vorzugsweise größer als 30 °C und besonders bevorzugt größer als 40 °C ist.

**26.** Polymer nach einem der vorhergehenden Ansprüche, **dadurch gekennzeichnet, dass** die Zwischensequenz eine Glasübergangstemperatur aufweist, die zwischen den Glasübergangstemperaturen der ersten und der zweiten Sequenz liegt.

**27.** Polymer nach einem der vorhergehenden Ansprüche, **dadurch gekennzeichnet, dass** es einen Polydispersitätsindex von größer oder gleich 2,8 aufweist.

**28.** Polymer nach einem der vorhergehenden Ansprüche, **dadurch gekennzeichnet, dass** es einen Polydispersitätsindex im Bereich von 2,8 bis 6 aufweist.

**29.** Polymer nach einem der vorhergehenden Ansprüche, dessen gewichtsmittlere Molmasse (Mw) kleiner oder gleich 300000 ist.

**30.** Polymer nach einem der vorhergehenden Ansprüche, dessen gewichtsmittlere Molmasse (Mw) im Bereich von 35000 bis 200000 und noch besser im Bereich von 45000 bis 150000 liegt.

**31.** Polymer nach einem der vorhergehenden Ansprüche, dessen zahlenmittlere Molmasse (Mn) kleiner oder gleich 70000 ist.

**32.** Polymer nach einem der vorhergehenden Ansprüche, dessen zahlenmittlere Molmasse (Mn) im Bereich von 10000 bis 60000 und noch besser im Bereich von 12000 bis 50000 liegt.

**33.** Polymer nach einem der vorhergehenden Ansprüche, **dadurch gekennzeichnet, dass** es in einer Menge der wirksamen Substanz von mindestens 1 Gew.-% in Wasser oder einem Gemisch von Wasser und geradkettigen oder verzweigten niederen Monoalkoholen mit 2 bis 5 Kohlenstoffatomen bei Raumtemperatur (25 °C) ohne pH-Änderung unlöslich ist.

**34.** Polymer nach einem der vorhergehenden Ansprüche, **dadurch gekennzeichnet, dass** es kein Elastomer ist.

**35.** Polymer nach einem der vorhergehenden Ansprüche, **dadurch gekennzeichnet, dass** es ein filmbildendes lineares ethylenisches Sequenzpolymer ist.

**36.** Verfahren zur Herstellung eines Polymers nach einem der vorhergehenden Ansprüche, das die folgenden Schritte umfasst:

- ein Teil des Polymerisationslösemittels wird in einen geeigneten Reaktor gegeben und erwärmt, bis die geeignete Polymerisationstemperatur erreicht ist,
- nach Erreichen dieser Temperatur werden die Monomere, die die erste Sequenz aufbauen, in Gegenwart eines Polymerisationsstarters zugegeben,
- nach einer Zeit T, die einem maximalen Umwandlungsgrad von 90 % entspricht, werden die Monomere, die die zweite Sequenz aufbauen, und der andere Teil des Polymerisationslösemittels zugegeben,
- das Gemisch wird eine Zeitspanne T' reagieren gelassen, wonach das Gemisch abgekühlt wird,
- man erhält das Polymer gelöst in dem Polymerisationslösemittel.

**37.** Verfahren nach Anspruch 36, **dadurch gekennzeichnet, dass** die Polymerisationstemperatur im Bereich von 60 bis 120 °C liegt.

**38.** Kosmetische Zusammensetzung, **dadurch gekennzeichnet, dass** sie ein Polymer nach einem der Ansprüche 1 bis 35 enthält.

**39.** Kosmetische Zusammensetzung nach Anspruch 38, **dadurch gekennzeichnet, dass** sie als wirksame Substanz ausgedrückt 0,1 bis 60 Gew.-% Polymer, vorzugsweise 5 bis 50 Gew.-% und noch bevorzugter 10 bis 40 Gew.-% enthält.

**40.** Zusammensetzung nach einem der Ansprüche 38 bis 39, **dadurch gekennzeichnet, dass** sie ein physiologisch akzeptables Medium enthält.

**41.** Zusammensetzung nach einem der Ansprüche 38 bis 40, **dadurch gekennzeichnet, dass** das physiologisch akzeptables Medium ein hydrophiles Medium enthält, das Wasser und Gemische von Wasser und einem oder mehreren hydrophilen organischen Lösemittel(n) umfasst, wie Alkohole und insbesondere lineare oder verzweigte, niedere Monoalkohole mit 2 bis 5 Kohlenstoffatomen, wie Ethanol, Isopropanol oder n-Propanol, Polyole, wie Glycerin, Diglycerin, Propylenglycol, Sorbit, Pentylenglycol, und Polyethylenglycole.

**42.** Zusammensetzung nach einem der Ansprüche 38 bis 41, **dadurch gekennzeichnet, dass** sie ferner eine Fettphase enthält, die aus bei Raumtemperatur flüssigen oder festen Fettsubstanzen tierischer, pflanzlicher, mineralischer oder synthetischer Herkunft zusammengesetzt ist.

**43.** Zusammensetzung nach einem der Ansprüche 38 bis 42, **dadurch gekennzeichnet, dass** sie ferner ein oder mehrere kosmetisch akzeptable organische Lösemittel enthält.

**44.** Zusammensetzung nach einem der Ansprüche 38 bis 43, **dadurch gekennzeichnet, dass** sie ferner ein oder mehrere Hilfsstoffe für die Filmbildung enthält, die unter den Weichmachern und Koaleszenzmitteln ausgewählt sind.

**45.** Zusammensetzung nach einem der Ansprüche 38 bis 44, **dadurch gekennzeichnet, dass** sie ferner ein oder mehrere Farbmittel enthält, die unter den wasserlöslichen Farbstoffen und pulverförmigen Farbstoffen, wie Pigmenten, Perlglanzpigmenten und Pailletten ausgewählt sind.

**46.** Zusammensetzung nach einem der Ansprüche 38 bis 45, **dadurch gekennzeichnet, dass** sie ferner Füllstoffe enthält.

**47.** Zusammensetzung nach einem der Ansprüche 38 bis 46, **dadurch gekennzeichnet, dass** sie ferner einen oder mehrere Bestandteil(e) enthält, die unter den Vitaminen, Verdickungsmitteln, Spurenelementen, beruhigenden Stoffen, Maskierungsmitteln, Parfums, Alkalisierungsmitteln oder Ansäuerungsmitteln, Konservierungsmitteln, Sonnenschutzfiltem, grenzflächenaktiven Stoffen, Antioxidantien, Wirkstoffen gegen Haarausfall, Antischuppenmitteln, Treibmitteln und deren Gemischen ausgewählt sind.

**48.** Zusammensetzung nach einem der Ansprüche 38 bis 47, **dadurch gekennzeichnet, dass** sie als Suspension, Dispersion, Lösung, Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), als Creme, Paste, Schaum, Vesikeldispersion insbesondere von ionischen oder nichtionischen Lipiden, zweiphasige oder mehrphasige Lotion, Pulver, Paste, insbesondere weiche Paste oder wasserfreie Paste, vorliegt.

**49.** Zusammensetzung nach einem der Ansprüche 38 bis 48, **dadurch gekennzeichnet, dass** es sich um eine Zusammensetzung zum Schminken oder zur Pflege von Keratinsubstanzen handelt.

**50.** Zusammensetzung nach einem der Ansprüche 38 bis 49, **dadurch gekennzeichnet, dass** es sich um eine Zusammensetzung für die Haarbehandlung handelt.

**51.** Zusammensetzung nach einem der Ansprüche 38 bis 49, **dadurch gekennzeichnet, dass** es sich um einen Nagellack handelt.

**52.** Zusammensetzung nach einem der Ansprüche 38 bis 49, **dadurch gekennzeichnet, dass** es sich um ein Produkt zum Schminken der Lippen handelt.

**53.** Zusammensetzung nach einem der Ansprüche 38 bis 49, **dadurch gekennzeichnet, dass** es sich um ein Produkt zum Schminken der Augen handelt.

**54.** Zusammensetzung nach einem der Ansprüche 38 bis 49, **dadurch gekennzeichnet, dass** es sich um ein Schminkprodukt für den Teint handelt.

**55.** Zusammensetzung nach Anspruch 54, **dadurch gekennzeichnet, dass** das Sequenzpolymer eine Sequenz mit einer Tg im Bereich von 50 bis 100 °C und eine Sequenz mit einer Tg im Bereich von -100 bis 0 °C enthält.

**56.** Zusammensetzung nach Anspruch 54, **dadurch gekennzeichnet, dass** das Sequenzpolymer ausgewählt ist unter:

(i) einem Sequenzpolymer, das umfasst:

- eine erste Sequenz mit einer Tg von größer oder gleich 40 °C, beispielsweise im Bereich von 85 bis 115 °C, die ein Isobornylacrylat/Isobutylmethacrylat-Copolymer ist;
- eine zweite Sequenz mit einer Tg von kleiner oder gleich 20 °C, beispielsweise im Bereich von -85 bis -55 °C, die ein 2-Ethylhexylacrylat-Homopolymer ist; und
- eine Zwischensequenz, die ein statistisches Copolymer Isobornylacrylat/Isobutylmethacrylat/2-Ethylhexylacrylat ist;

(ii) einem Sequenzpolymer, das umfasst:

- eine erste Sequenz mit einer Tg von größer oder gleich 40 °C, beispielsweise im Bereich von 60 bis 90 °C, die ein Isobornylacrylat/Isobutylmethacrylat-Copolymer ist;
- eine zweite Sequenz mit einer Tg von kleiner oder gleich 20 °C, beispielsweise im Bereich von -35 bis -5 °C, die ein Isobutylacrylat-Homopolymer ist; und
- eine Zwischensequenz, die ein statistisches Copolymer Isobornylacrylat/Isobutylmethacrylat/Isobutylacrylat ist.

**57.** Zusammensetzung nach einem der Ansprüche 38 bis 56, **dadurch gekennzeichnet, dass** sie ein ergänzendes filmbildendes Polymer enthält.

**58.** Zusammensetzung nach einem der Ansprüche 38 bis 57, **dadurch gekennzeichnet, dass** sie eine feste Fettsubstanz enthält, die unter den Wachsen, pastösen Fettsubstanzen, Gummis und deren Gemischen ausgewählt ist.

**59.** Kosmetisches Verfahren zum Schminken oder für die Pflege von Keratinsubstanzen, das umfasst, eine kosmetische Zusammensetzung nach einem der Ansprüche 38 bis 58 auf die Keratinsubstanzen aufzutragen.

**60.** Verwendung eines Polymers nach einem der Ansprüche 1 bis 37 in einer kosmetischen Zusammensetzung, als Stoff, um die Beständigkeit der Zusammensetzung zu verbessern.

**61.** Verwendung eines Polymers nach einem der Ansprüche 1 bis 3 in einer kosmetischen Zusammensetzung, die bezüglich der Beständigkeit bessere Eigenschaften aufweist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2809306 A